# EUROPEAN PATENT APPLICATION

(11) **EP 4 717 149 A1**
(43) Date of publication of application: **01.04.2026**
(21) Application number: 25203257.8
(22) Date of filing: 19.09.2025
(51) Int. Cl.: A61B 3/00, A61B 3/12

(54) **OPHTHALMIC APPARATUS**

(30) Priority: 26.09.2024 JP 2024167786
(71) Applicant: Topcon Corporation, Tokyo 174-8580 (JP)
(72) Inventor: KASEYAMA, Yuta, Tokyo, 174-8580 (JP); KOBAYASHI, Mariko, Tokyo, 174-8580 (JP); NAKAJIMA, Masashi, Tokyo, 174-8580 (JP); SUZUKI, Masaya, Tokyo, 174-8580 (JP)
(74) Representative: Vossius & Partner Patentanwälte Rechtsanwälte mbB

(57) **Abstract**

An ophthalmic apparatus includes a first scanning optical system, a dividing filter, a descanning optical system, and one or more black spot plates. The first scanning optical system is configured to deflect slit-shaped illumination light from the light source unit using the scanning mirror to guide the deflected illumination light to the eye to be examined through the objective lens. The dividing filter is positioned at a position substantially conjugate optically to the pupil between the light source unit and the scanning mirror. In the dividing filter, two or more illumination openings and a single light receiving opening, or a single illumination opening and two or more light receiving openings are formed. The descanning optical system is configured to guide returning light of the illumination light from the eye to be examined to an imaging element. Here, the returning light is guided through the objective lens and the scanning mirror and passes through the light receiving opening. The one or more black spot plates are positioned at positions where the illumination light reflected by the objective lens is imaged in the first scanning optical system on an optical path between the objective lens and the scanning mirror.

## Description

The disclosure relates to an ophthalmic apparatus.

Ophthalmic apparatuses used for screening or treatment of eye diseases are expected to be capable of easily photographing (observing) a fundus of an eye to be examined with a wide field of view. As such ophthalmic apparatuses, scanning laser ophthalmoscopes (SLOs) are known. SLO is an apparatus configured to form an image of the fundus by scanning the fundus with light to detect returning light of the light with a light receiving device.

Japanese Unexamined Patent Application Publication (Translation of PCT Application) No. 2017-526474 discloses a method of acquiring clear fundus images of the eye to be examined by scanning a retina with slit-shaped light using a projection diaphragm and a confocal diaphragm which are positioned at positions substantially conjugate optically to the retina.

One aspect of some embodiments is an ophthalmic apparatus including a first scanning optical system, a dividing filter, a descanning optical system, and one or more black spot plates. The first scanning optical system includes an objective lens and a scanning mirror positioned at a position substantially conjugate optically to a pupil of an eye to be examined, and is configured to deflect slit-shaped illumination light from a light source unit using the scanning mirror to guide the deflected illumination light to the eye to be examined through the objective lens. The dividing filter is positioned at a position substantially conjugate optically to the pupil between the light source unit and the scanning mirror. In the dividing filter, two or more illumination openings and a single light receiving opening, or a single illumination opening and two or more light receiving openings are formed. The descanning optical system is configured to guide returning light of the illumination light from the eye to be examined to an imaging element. The returning light is guided through the objective lens and the scanning mirror and passes through the light receiving opening. The imaging element is positioned at a position substantially conjugate optically to a photographed site of the eye to be examined. The one or more black spot plates are positioned at positions where the illumination light reflected by the objective lens is imaged in the first scanning optical system on an optical path between the objective lens and the scanning mirror.
FIG. 1 is a schematic diagram illustrating an example of a configuration of an optical system of an ophthalmic apparatus according to a first embodiment.
FIG. 2 is a schematic diagram for explaining the configuration of the optical system of the ophthalmic apparatus according to the first embodiment.
FIG. 3 is a schematic diagram for explaining the configuration of the optical system of the ophthalmic apparatus according to the first embodiment.
FIG. 4 is a schematic diagram illustrating an example of a configuration of a processing system of the ophthalmic apparatus according to the first embodiment.
FIG. 5 is a schematic diagram illustrating an example of the configuration of the processing system of the ophthalmic apparatus according to the first embodiment.
FIG. 6 is a flowchart illustrating an example of an operation of the ophthalmic apparatus according to the first embodiment.
FIG. 7A is a schematic diagram for explaining the operation of the ophthalmic apparatus according to the first embodiment.
FIG. 7B is a schematic diagram for explaining the operation of the ophthalmic apparatus according to the first embodiment.
FIG. 8 is a schematic diagram illustrating an example of a configuration of an optical system of the ophthalmic apparatus according to a second embodiment.
FIG. 9 is a schematic diagram illustrating an example of a configuration of an optical system according to a first modification example of the first embodiment or the second embodiment.
FIG. 10 is a schematic diagram illustrating an example of a configuration of an optical system according to a second modification example of the first embodiment or the second embodiment.
FIG. 11 is a schematic diagram illustrating an example of a configuration of an optical system according to a third modification example of the first embodiment or the second embodiment.
FIG. 12 is a schematic diagram illustrating an example of a configuration of an optical system according to a fourth modification example of the first embodiment or the second embodiment.
FIG. 13A is a schematic diagram for explaining a conventional ophthalmic apparatus.
FIG. 13B is a schematic diagram for explaining the conventional ophthalmic apparatus.
FIG. 14A is a schematic diagram for explaining the conventional ophthalmic apparatus.
FIG. 14B is a schematic diagram for explaining the conventional ophthalmic apparatus.
FIG. 15 is a schematic diagram for explaining the conventional ophthalmic apparatus.
FIG. 16 is a schematic diagram for explaining the conventional ophthalmic apparatus.

### DETAILED DESCRIPTION

In the ophthalmic apparatuses capable of photographing an intraocular site such as a fundus, illumination light is incident into the eye through a pupil, and reflected light (returning light, fundus reflected light) of the illumination light from the fundus is emitted through the pupil. Therefore, in the ophthalmic apparatuses, an image of an illumination opening, through which the illumination light passes, and an image of a light receiving opening (photographing opening), through which the reflected light passes, are separated on a pupil conjugate plane that is approximately conjugate optically to the pupil of the eye to be examined.

However, in the ophthalmic apparatus disclosed in Japanese Unexamined Patent Application Publication (Translation of PCT Application) No. 2017-526474 is configured so that a single illumination opening and a single light receiving opening are formed on the pupil conjugate plane. In this case, when the eye to be examined is an eye with microcoria (small pupil), image quality of the acquired image may deteriorate for the following reasons.

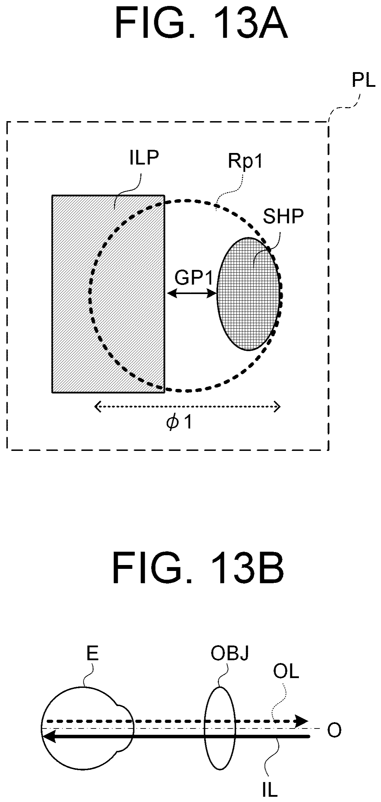

FIG. 13A and FIG. 13B show schematic diagrams illustrating a case of photographing the fundus of the eye to be examined with a normal pupil diameter, in the conventional ophthalmic apparatus disclosed in Japanese Unexamined Patent Application Publication (Translation of PCT Application) No. 2017-526474. FIG. 13A schematically represents the image of the illumination opening and the image of the light receiving opening in the conventional ophthalmic apparatus. FIG. 13B schematically represents the illumination light incident on the eye to be examined and the reflected light in a state shown in FIG. 13A.

In the ophthalmic apparatus described above, a separation diaphragm is positioned at a position substantially conjugate optically to a pupil of the eye E to be examined, in order to completely separate illumination light flux incident into the eye through the pupil and reflection light flux emitted from the eye through the pupil. In the separation diaphragm, an illumination opening, through which the illumination light flux IL passes, and a light receiving opening, through which the reflection light flux OL from the fundus passes, are formed. Thereby, an image ILP of the illumination opening and an image SHP of the light receiving opening are formed on a pupil conjugate plane PL (see FIG. 13A).

In fundus photography of the eye E to be examined, the illumination light is entered through the pupil, and the reflected light is emitted through the pupil. Therefore, on the pupil conjugate plane PL, the image ILP (at least a part thereof) of the illumination opening and the image SHP (at least a part thereof) of the light receiving opening must be put within a pupil division range Rp1 corresponding to the pupil diameter φ1 of the eye E to be examined (FIG. 13A). In this case, a gap between the image of the illumination opening and the image of the light receiving opening (gap between the illumination opening and the light receiving opening on the separation diaphragm) becomes a predetermined gap length GP1 (> 0). Here, the gap is a distance (shortest distance) between the image of the illumination opening and the image of the light receiving opening in an arrangement direction of the image of the illumination opening and the image of the light receiving opening.

When the gap is the predetermined gap length GP1 as shown in FIG. 13A, the illumination light flux IL is transmitted through an objective lens OBJ arranged on the optical axis O, and is guided to the fundus through the pupil of the eye E to be examined, as shown in FIG. 13B. The reflection light flux OL of the illumination light flux IL reflected on the fundus is emitted from the eye E to be examined through the pupil, and is transmitted through the objective lens OBJ to guide it to a light receiving optical path (photographing optical path).

In contrast, when the eye E to be examined is an eye with microcoria, the gap described above must be shortened.

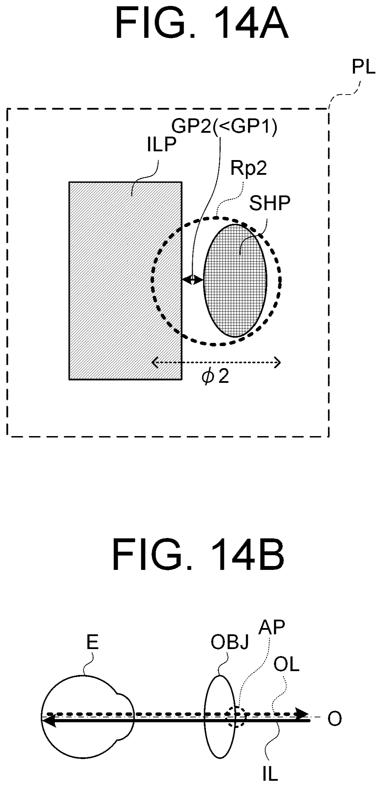

FIG. 14A and FIG. 14B show schematic diagrams illustrating a case of photographing the fundus of the eye to be examined with a microcoria, in the conventional ophthalmic apparatus disclosed in Japanese Unexamined Patent Application Publication (Translation of PCT Application) No. 2017-526474. FIG. 14A schematically represents the image of the illumination opening and the image of the light receiving opening in the conventional ophthalmic apparatus. FIG. 14B schematically represents the illumination light incident on the eye to be examined and the reflected light in a state shown in FIG. 14A. In FIG. 14A and FIG. 14B, like parts are designated by like reference numerals as in FIG. 13A and FIG. 13B and repetitious description of such parts may not be provided.

In case that the eye E to be examined is an eye with a microcoria, on the pupil conjugate plane PL, the image ILP (at least a part thereof) of the illumination opening and the image SHP (at least a part thereof) of the light receiving opening must be put within a pupil division range Rp2 corresponding to the pupil diameter φ2 of microcoria (FIG. 14A). In this case, a gap between the image of the illumination opening and the image of the light receiving opening becomes a predetermined gap length GP2 (< GP1).

When the gap is the predetermined gap length GP2 as shown in FIG. 14A, the illumination light flux IL passes near the optical axis of the objective lens OBJ, as shown in FIG. 14B. As a result, reflection at a lens apex AP on the optical axis O of the objective lens OBJ (center ghost) is likely to enter the light receiving optical path. Thereby, a flare occurs as an artifact, which increases the possibility of complete loss of at least some of the fundus information in the photographing region.

In this case, by arranging a black spot plate at a position, which is substantially conjugate optically to the lens apex AP of the objective lens OBJ on an illumination optical path, the center ghost caused by reflection from the lens apex AP can be prevented.

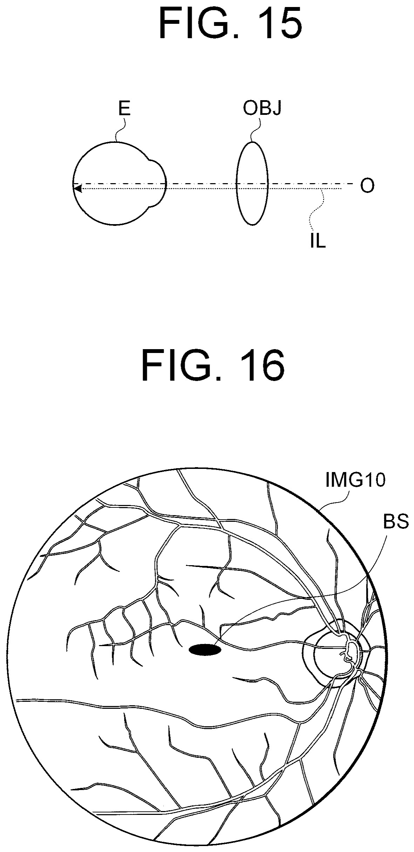

FIG. 15 and FIG. 16 show schematic explanatory diagrams when the black spot plate is arranged in the conventional ophthalmic apparatus disclosed in Japanese Unexamined Patent Application Publication (Translation of PCT Application) No. 2017-526474. FIG. 15 schematically represents the illumination light incident on the eye to be examined and the reflected light in a state where the black spot plate is arranged. FIG. 16 schematically represents the fundus image of the eye E to be examined acquired in the conventional ophthalmic apparatus in a state where the black spot plate is arranged. In FIG. 15, like reference numerals designate like parts as in FIG. 13A or FIG. 13B. The same description may not be repeated.

In case that, as described above, the black spot plate is arranged at a position, which is substantially conjugate optically to the lens apex AP of the objective lens OBJ on an illumination optical path, the illumination light passing through the lens apex AP of the objective lens OBJ is shielded by the black spot plate. As a result, the illumination light does not reach the fundus near the lens optical axis of the objective lens OBJ. In this case, as shown in FIG. 16, a black spot shadow BS caused by the black spot plate is depicted in the acquired fundus image IMG10. A region in which the black spot shadow BS is depicted is in a state of blackout (state in which gradation information is lost). Thus, the region becomes a region in which the fundus information as an intraocular information representing the morphology of the eye is completely lost.

As described above, in the conventional ophthalmic apparatus disclosed in Japanese Unexamined Patent Application Publication (Translation of PCT Application) No. 2017-526474, the image quality of the fundus image deteriorates due to the occurrence of flare or the depiction of black spot shadow. As a result, this causes a problem in which at least some of the fundus information in the photographing region area is lost. This type of problem occurs not only when the photographed site is the fundus, but also when photographing the interior of the eye to be examined.

According to embodiments according to the present invention, a new technique for suitably performing small pupil photography without completely losing intraocular information can be provided.

Referring now to the drawings, exemplary embodiments of an ophthalmic apparatus according to the present invention are described below. Any of the contents of the documents cited in the present specification and arbitrary known techniques may be applied to the embodiments below.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

An ophthalmic apparatus according to embodiments includes a scanning optical system (first scanning optical system), a descanning optical system, a dividing filter (pupil division filter), and a one or more black spot plates.

The scanning optical system includes an objective lens and a scanning mirror positioned at a position substantially conjugate optically to a pupil of an eye to be examined (subject's eye). Here, the position substantially conjugate optically to the pupil of the eye to be examined includes a pupil conjugate position conjugate optically to the pupil of the eye to be examined or the vicinity of the pupil conjugate position. The scanning optical system is configured to deflect slit-shaped (line-shaped) illumination light from a light source unit using the scanning mirror to irradiate the deflected illumination light onto the eye to be examined through the objective lens. By sequentially moving an irradiated region of the illumination light, a predetermined illumination range including a desired photographed site (for example, fundus) in the eye to be examined is scanned with the illumination light.

The descanning optical system is configured to descan returning light (reflected light) of the illumination light, that has been guided through the objective lens from the eye to be examined, using the scanning mirror to guide the descanned returning light (specifically, returning light that has passed through a light receiving opening further described below) to an imaging element. The imaging element (imaging surface) is positioned at a position substantially conjugate optically to a photographed site of the eye to be examined. Here, in case that the photographed site is the fundus, the position substantially conjugate optically to the fundus of the eye to be examined includes a fundus conjugate position conjugate optically to the pupil of the eye to be examined or the vicinity of the fundus conjugate position.

The dividing filter is configured to optically divide (separate) an optical path of the descanning optical system from an optical path of the scanning optical system. The dividing filter is positioned at the pupil conjugate position or in the vicinity of the pupil conjugate position. In some embodiments, the dividing filter is positioned at the pupil conjugate position or in the vicinity of the pupil conjugate position on an optical path between the light source unit and the scanning mirror. In the dividing filter, two or more illumination openings and a single light receiving opening (photographing opening), or a single illumination opening and two or more light receiving openings are formed.

In case that the two or more illumination openings and the single light receiving opening are formed in the dividing filter, the illumination light that has passed through the two or more illumination openings is guided to the photographed site of the eye to be examined, and the returning light from the photographed site that has passed through the single light receiving opening is guided to the descanning optical system.

In case that the single illumination opening and the two or more light receiving openings are formed in the dividing filter, the illumination light that has passed through the single illumination opening is guided to the photographed site of the eye to be examined, and the returning light from the photographed site that has passed through the two or more light receiving openings is guided to the descanning optical system.

The one or more black spot plates are positioned at or in the vicinity of the positions where the illumination light reflected by the objective lens is imaged in the scanning optical system on an optical path between the objective lens and the scanning mirror. In some embodiments, the one or more black spot plates are positioned at positions (lens apex conjugate positions) conjugate optically to the lens apex of the objective lens or in the vicinity of lens apex conjugate positions on the optical axis of the scanning optical system. In some embodiments, the one or more black spot plates are positioned at positions or in the vicinity of the positions where the illumination light reflected by the objective lens is imaged in the scanning optical system on the optical path between the objective lens and the scanning mirror, among the lens apex conjugate positions or the vicinity of the lens apex conjugate positions.

In some embodiments, the one or more black spot plates are positioned at or in the vicinity of positions where the illumination light reflected by the objective lens is imaged in the scanning optical system with the position of an illumination diaphragm as an object point. Here, the position of the illumination diaphragm is a position of the illumination opening (the two or more illumination openings or the single illumination opening) formed in the dividing filter.

In some embodiments, the one or more black spot plates are positioned at at least one of a position where the illumination light reflected on an anterior surface of the objective lens is imaged in the scanning optical system, or a position where the illumination light reflected on a posterior surface of the objective lens is imaged in the scanning optical system.

In some embodiments, the objective lens includes one or more doublets configured by sticking two or more lenses. In this case, the one or more black spot plates are positioned at positions where the illumination light reflected on one or more sticking surfaces of the one or more doublets is imaged in the scanning optical system.

As described above, two or more black spot shadows are depicted in the image of the eye to be examined obtained by illuminating the photographed site of the eye to be examined with the illumination light that has passed through the two or more illumination openings or the image of the eye to be examined obtained by receiving the returning light from the photographed site that has passed through the two or more light receiving openings. Here, the two or more black spot shadows correspond to the two or more illumination openings or the two or more light receiving openings, respectively. Therefore, each black spot shadow includes gradation information (intraocular information) as fundus information obtained by illuminating with illumination light that has passed through the remaining illumination openings excluding the corresponding illumination opening. Or, each black spot shadow includes gradation information obtained by the returning light that has passed through the remaining light receiving openings excluding the corresponding light receiving opening. As a result, each of the two or more black spot shadows is not in a state of blackout, and a region of each black spot shadow includes gradation information corresponding to the light quantity of the illumination light that has passed through the remaining illumination openings or the light quantity of the returning light that has passed through the remaining light receiving openings.

For example, by performing predetermined black spot shadow correction processing on the obtained image of the eye to be examined, complete loss of the gradation information can be avoided. Thereby, even when the gap needs to be shortened, such as when the eye to be examined is an eye with microcoria, it becomes possible to acquire images including the intraocular information of the eye to be examined and make appropriate diagnoses for images of the eyes with microcoria.

A method of controlling the ophthalmic apparatus according to the embodiments includes one or more steps for realizing the processing executed by one or more processors (computers) in the ophthalmic apparatus according to the embodiments. A program according to the embodiments causes the one or more processors to execute each step of the method of controlling the ophthalmic apparatus according to the embodiments. In other words, the program according to the embodiments is a computer program comprising instructions which, when the program is executed by a computer, cause the computer to carry out the method of controlling the ophthalmic apparatus according to the embodiments. A recording medium (storage medium) according to the embodiments is any computer readable non-transitory recording medium (storage medium) on which the program according to the embodiments is recorded. The recording medium may be an electronic medium using magnetism, light, magneto-optical, semiconductor, or the like. Typically, the recording medium is a magnetic tape, a magnetic disk, an optical disk, a magneto-optical disk, a flash memory, a solid state drive, or the like. Examples of magnetic disk include a magnetic storage media such as a hard disk, a floppy (registered trademark) disks, and a ZIP. Examples of the magneto-optical disk include CD-ROM, DVD-RAM, DVD-ROM, and MO. Further, the program may be transmitted and received through a network such as the Internet, LAN, etc.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the ophthalmic apparatus according to the embodiments is a fundus photographing apparatus for photographing the fundus as the photographed site will be mainly described. However, the following embodiments can be applied to the ophthalmic apparatus in which the photographed site is a site other than the fundus.

Hereinafter, the position conjugate optically to the fundus of the eye to be examined may be referred to as the "fundus conjugate position", and the position substantially conjugate optically to the fundus of the eye to be examined may be referred to as the "fundus conjugate position or the vicinity of the fundus conjugate position". In the same way, the position conjugate optically to the pupil of the eye to be examined may be referred to as the "pupil conjugate position", and the position substantially conjugate optically to the pupil of the eye to be examined may be referred to as the "pupil conjugate position or the vicinity of the pupil conjugate position".

### <First embodiment>

### [Optical system]

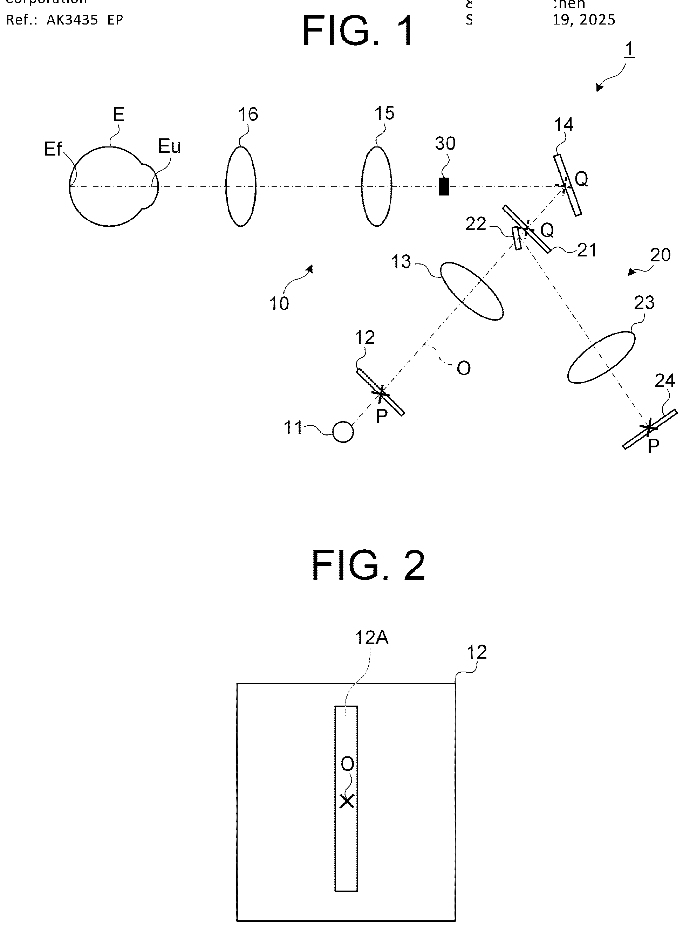

FIG. 1 illustrates an example of a configuration of an optical system of an ophthalmic apparatus according to a first embodiment.

An ophthalmic apparatus 1 according to the first embodiment is configured to scan a fundus Ef of an eye E to be examined with slit-shaped illumination light, and to acquire a fundus image of the eye E to be examined by sequentially receiving returning light of the illumination light from the fundus Ef. Here, the returning light from the fundus Ef is scattered light (reflected light) of the illumination light from the fundus Ef onto which the illumination light incident on the eye E to be examined is irradiated. In some embodiments, the returning light from the eye E to be examined includes scattered light (reflected light) of the illumination light from the fundus Ef, and fluorescence and its scattered light using the illumination light incident on the eye E to be examined as excitation light. In FIG. 1, a position conjugate optically to the fundus Ef of the eye E to be examined is illustrated as a fundus conjugate position P, and a position substantially conjugate optically to a pupil Eu of the eye E to be examined is illustrated as a pupil conjugate position Q.

The ophthalmic apparatus 1 includes a scanning optical system 10 including a scanning mirror 14, a descanning optical system 20, a dividing filter 21, and an imaging element 24.

The scanning optical system 10 is configured to irradiate slit-shaped illumination light from a light source unit onto the fundus Ef of the eye E to be examined, as an illumination optical system. The descanning optical system 20 is configured to guide returning light of the illumination light from the fundus Ef that has traveled through an optical path of the scanning optical system 10 to the imaging element 24. In FIG. 1, the scanning mirror 14 and the dividing filter 21 are positioned on an optical axis O of the scanning optical system 10. The scanning mirror 14 scans the fundus Ef with the illumination light by deflecting the slit-shaped illumination light. The dividing filter 21 divides the illumination light and the returning light of the illumination light on a pupil conjugate plane (eye conjugate plane), and guides the divided returning light to the imaging element 24.

The scanning optical system 10 includes, in addition to the scanning mirror 14, a light source 11, a slit diaphragm 12, a relay lens 13, a black spot plate 30, a relay lens 15, and an objective lens 16.

### (Light source 11)

The light source 11 outputs light in the wavelength range of the visible region or the infrared region. For example, the light source 11 includes an LED (Light Emitting Diode), or an LD (Laser Diode).

In some embodiments, the light source 11 includes two or more light sources that generate light in different wavelength ranges, and can emit light synthesized from light generated simultaneously from the two or more light sources, or can emit light generated from any one of the two or more light sources.

### (Slit diaphragm 12)

In the slit diaphragm 12, one or more slits (slit-shaped openings) are formed. The slit diaphragm 12 (specifically, one or more slits) is positioned at a position (fundus conjugate position or the vicinity of the fundus conjugate position) substantially conjugate optically to the fundus Ef of the eye E to be examined. In the following, it is assumed that a single slit is formed in the slit diaphragm 12.

FIG. 2 shows an explanatory diagram of the slit diaphragm 12 in FIG. 1. FIG. 2 schematically represents an example of a configuration of the slit diaphragm 12 when viewed from the optical axis O of the scanning optical system 10.

In the slit diaphragm 12, a slit 12A through which the optical axis O passes is formed. By uniformly irradiating the light from the light source 11 onto the slit diaphragm 12, the light passing through the slit 12A is emitted as the slit-shaped illumination light as a secondary light source.

The slit 12A has a long rectangular shape with the long side being a slit direction. The shape of the slit 12A defines a shape of an irradiated region (irradiated pattern shape) of the illumination light on the fundus Ef of the eye E to be examined. By deflecting the light in at least the direction intersecting the slit direction using the scanning mirror 14 described below, the returning light from the irradiated region moved on the fundus Ef is sequentially received.

In some embodiments, the slit diaphragm 12 is configured so that a slit shape of the slit 12A can be changed. For example, the slit shape of the slit 12A can be changed according to at least one of a fundus shape of the eye E to be examined (shape of the photographed site), a dioptric power (refractive power) of the eye E to be examined, or the shape of the irradiated region of the illumination light on the fundus Ef. With this, the deterioration of the fundus image of the eye E to be examined caused by distortion of the irradiated region of the illumination light on the fundus Ef can be easily prevented. Such a slit diaphragm 12 is realized, for example, by a liquid crystal shutter.

In some embodiments, the slit diaphragm 12 is configured to tilt in a traveling direction of the illumination light or a direction intersecting the traveling direction relative to the optical axis O. For example, an angle (tilt angle) of the slit 12A relative to the optical axis O can be changed according to at least one of a fundus shape of the eye E to be examined (shape of the photographed site), a dioptric power of the eye E to be examined, or the shape of the irradiated region of the illumination light on the fundus Ef. The light source 11 may be configured to tilt in conjunction with the tilt of the slit diaphragm 12.

In some embodiments, a condenser lens is arranged between the light source 11 and the slit diaphragm 12. The condenser lens can increase light quantity of the light passing through the slit 12A, by condensing the light from the light source 11.

### (Relay lens 13)

The relay lens 13 transmits the illumination light passing through the slit 12A formed in the slit diaphragm 12 to guide the illumination light to the dividing filter 21.

In some embodiment, the relay lens 13 is configured to be capable of moving in a direction of the optical axis O, as a focusing lens. By moving the relay lens 13 in the direction of the optical axis O, the slit diaphragm 12 (slit 12A) can be arranged at the fundus conjugate position or in the vicinity of the fundus conjugate position.

In some embodiment, the slit diaphragm 12 is configured to be capable of moving in the direction of the optical axis O. In some embodiment, each of the slit diaphragm 12 and the relay lens 13 is configured to be capable of moving in conjunction with each other or independently.

### (Scanning mirror 14)

The scanning mirror 14 is positioned on the optical axis O of the scanning optical system 10, as an optical scanner. The scanning mirror 14 (deflected surface of scanning mirror, scanning surface of scanning mirror) is positioned at a position (pupil conjugate position or in the vicinity of the pupil conjugate position) substantially conjugate optically to the pupil Eu of the eye E to be examined. The scanning mirror 14 one-dimensionally deflects the illumination light in an intersecting direction (orthogonal direction) of the slit direction of the slit 12A, for example.

The scanning mirror 14 includes, for example, a galvanometer scanner, a MEMS (Micro Electro Mechanical System) scanner, a polygon mirror, or a resonant scanner. For example, the scanning mirror 14 includes the galvanometer scanner that deflects the illumination light within a predetermined deflection angular range with reference to a predetermined deflection reference angle direction.

In some embodiments, the scanning mirror 14 two-dimensionally deflects the illumination light in two-dimensional directions including the intersecting direction (orthogonal direction) of the slit direction of the slit 12A. For example, the scanning mirror 14 includes a first scanning mirror and a second scanning mirror. The first scanning mirror deflects the illumination light so as to move the irradiated region on the fundus Ef in a horizontal direction orthogonal to the optical axis of the scanning optical system 10. The second scanning mirror deflects the illumination light deflected by the first scanning mirror so as to move the irradiated region on the fundus Ef in a vertical direction orthogonal to the optical axis of the scanning optical system 10.

### (Dividing filter 21)

Between the relay lens 13 and the scanning mirror 14, the dividing filter 21 is positioned.

The dividing filter 21 is positioned at the pupil conjugate position or in the vicinity of the pupil conjugate position. The dividing filter 21 divides (performs pupil division) the illumination light and the returning light of the illumination light on the pupil conjugate plane at the pupil conjugate position or in the vicinity of the pupil conjugate position. By positioning the dividing filter 21 in the vicinity of the deflected surface of the scanning mirror 14, the size of the optical system of the ophthalmic apparatus 1 can be reduced.

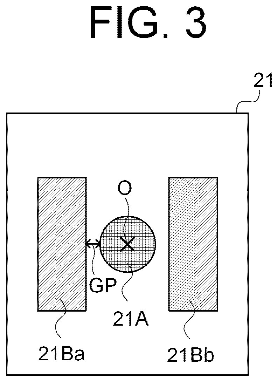

FIG. 3 shows an explanatory diagram of the dividing filter 21 in FIG. 1. FIG. 3 schematically represents an example of a configuration of the dividing filter 21 when viewed from the optical axis O of the scanning optical system 10.

In the dividing filter 21, the single light receiving opening is formed in a central region including a position corresponding to the optical axis O, and the two or more illumination openings are formed in a peripheral region eccentric to the optical axis O. It is assumed that, in the first embodiment, a single light receiving opening 21A and two illumination openings 21Ba and 21Bb are formed in the dividing filter 21.

The light receiving opening 21A is formed in the central region including the position corresponding to the optical axis O as an optical axis of the illumination light. The two illumination openings 21Ba and 21Bb are formed in the peripheral region eccentric to the optical axis O. In this case, the illumination opening 21Ba (21Bb) and the light receiving opening 21A are formed separated by a distance of the gap GP.

In FIG. 3, the shape of the light receiving opening 21A is a circular shape or a shape of an ellipse. However, the configuration according to the embodiments is not limited to the shape of the light receiving opening 21A. In addition, the shapes of the illumination openings 21Ba and 21Bb are rectangles (rectangular shapes, square shapes). However, the configuration according to the embodiments is not limited to the shape of the illumination opening 21Ba or 21Bb. Further, the shapes of the two or more illumination openings including the illumination openings 21Ba and 21Bb may be different from each other. Furthermore, the two or more illumination openings and the two or more light receiving openings are formed in the dividing filter 21.

In the dividing filter 21, on the pupil conjugate plane, the illumination light from the relay lens 13 passes through the illumination openings 21Ba and 21Bb, the returning light of the illumination light from the fundus Ef is descanned by the scanning mirror 14, and the descanned returning light passes through the light receiving opening 21A. The returning light that has passed through the light receiving opening 21A is guided to the descanning optical system 20.

### (Relay lens 15)

The relay lens 15 transmits the illumination light deflected by the scanning mirror 14 to guide the illumination light to the objective lens 16, and also transmits the returning light of the illumination light from the objective lens 16 to guide the returning light to the scanning mirror 14.

### (Objective lens 16)

The objective lens 16 guides the illumination light that has been transmitted through the relay lens 15 to the eye E to be examined to irradiate the fundus Ef through the pupil Eu, and also guides the returning light of the illumination light from the fundus Ef emitted through the pupil Eu to guide the returning light to the relay lens 15.

In some embodiments, the objective lens 16 includes two or more lenses. In some embodiments, the objective lens 16 includes one or more doublets configured by sticking two or more lenses.

### (Black spot plate 30)

The black spot plate 30 is positioned on the optical axis O between the objective lens 16 and the scanning mirror 14. The black spot plate 30 shields the illumination light reflected by the objective lens 16. On the optical axis O, one or more black spot plates 30 may be arranged.

Each of the one or more black spot plates 30 is positioned at or in the vicinity of a position ("objective lens reflected light imaging position") where the illumination light reflected by the objective lens 16 is imaged in the scanning optical system 10 on an optical path between the objective lens 16 and the scanning mirror 14.

In some embodiments, each of the one or more black spot plates 30 is positioned at a position (lens apex conjugate position) conjugate optically to the lens apex of the objective lens 16 or in the vicinity of the lens apex conjugate position on the optical axis O. In some embodiments, each of the one or more black spot plates 30 is positioned at the "objective lens reflected light imaging position" described above or in the vicinity of the "objective lens reflected light imaging position" on the optical path between the objective lens 16 and the scanning mirror 14 among the "objective lens reflected light imaging positions" or the vicinity of the "objective lens reflected light imaging positions".

In some embodiments, each of the one or more black spot plates 30 is positioned at or in the vicinity of a position where the illumination light reflected by the objective lens 16 is imaged in the scanning optical system 10 with the illumination openings 21Ba and 21Bb as object points.

In some embodiments, each of the one or more black spot plates 30 is positioned at at least one of a position where the illumination light reflected on an anterior surface of the objective lens 16 is imaged in the scanning optical system 10, or a position where the illumination light reflected on a posterior surface of the objective lens 16 is imaged in the scanning optical system 10. Here, the anterior surface of the objective lens 16 is a lens surface of the objective lens 16 on the side facing the eye E to be examined, and the posterior surface of the objective lens 16 is a lens surface on the light source 11 side.

As described above, in the case that the objective lens 16 includes the one or more doublets, each of the one or more black spot plates 30 is positioned at a position where the illumination light reflected on one or more sticking surfaces of the one or more doublets is imaged in the scanning optical system 10.

In some embodiments, by adjusting the curvature of the lens surface of the objective lens 16, etc. so that two or more positions where the reflected light of the illumination light by the objective lens 16 is imaged in the scanning optical system 10 described above are consolidated at a single position, a single black spot plate 30 can be arranged on the optical axis O. It should be noted that, in FIG. 1, the single black spot plate 30 is positioned between the relay lens 15 and the scanning mirror 14.

### (Branch mirror 22)

The branch mirror 22 may be positioned on the optical axis O between the relay lens 13 and the light receiving opening 21A formed in the dividing filter 21. In this case, the branch mirror 22 is positioned at the pupil conjugate position or in the vicinity of the pupil conjugate position.

The branch mirror 22 deflects the returning light of the illumination light from the fundus Ef that has passed through the light receiving opening 21A (returning light descanned by the scanning mirror 14) toward the descanning optical system 20.

### (Descanning optical system 20)

The descanning optical system 20 guides the returning light of the illumination light divided on the pupil conjugate plane by the dividing filter 21 to the imaging element 24, as a light receiving optical system. In FIG. 1, the descanning optical system 20 guides the returning light of the illumination light deflected by the branch mirror 22 (returning light of the illumination light divided by the dividing filter 21) to the imaging element 24.

The descanning optical system 20 includes a relay lens 23 and the imaging element 24. In some embodiments, the descanning optical system 20 further includes the branch mirror 22.

### (Relay lens 23)

The relay lens 23 transmits the returning light deflected by the branch mirror 22 to guide the returning light to the imaging element 24 (imaging surface).

In some embodiment, the relay lens 23 is configured to be capable of moving in an optical axis direction of the descanning optical system 20, as a focusing lens. By moving the relay lens 23 in the optical axis direction of the descanning optical system 20, the imaging element 24 (imaging surface) can be arranged at the fundus conjugate position or in the vicinity of the fundus conjugate position.

In some embodiment, the imaging element 24 is configured to be capable of moving in the optical axis direction of the descanning optical system 20.

### (Imaging element 24)

The imaging element 24 (imaging surface, light receiving surface, detection surface) can be arranged at the fundus conjugate position or in the vicinity of the fundus conjugate position. The imaging element 24 realizes the function of a one-dimensional image sensor or a two-dimensional image sensor as a pixelated photodetector.

The light flux (optical path) of the returning light of the illumination light descanned by the scanning mirror 14 is fixed in the descanning optical system 20. Therefore, the imaging element 24 can receive the returning light from the irradiated region of the illumination light on the fundus Ef by means of the one-dimensional image sensor (e.g., a line sensor) having a light receivable region (light-receptive region) in which light receiving elements are one-dimensionally arranged.

In case that the imaging element 24 is the two-dimensional image sensor, the imaging element 24 is configured to be capable of setting a light receivable region (light-receptive region) that can be virtually moved at the position substantially conjugate optically to the fundus Ef. In this case, the imaging element 24 can receive the returning light from the irradiated region of the illumination light on the fundus Ef in a predetermined light receivable region where the light receiving elements are one-dimensionally arranged in the imaging region where the light receiving elements are two-dimensionally arranged.

The imaging element 24 with such a configuration includes a CMOS (Complementary Metal Oxide Semiconductor) image sensor or CCD (Charge Coupled Device) image sensor.

In some embodiments, the descanning optical system 20 includes a confocal diaphragm positioned at a position substantially conjugate optically to the fundus Ef as a photographed site of the eye E to be examined. For example, the confocal diaphragm is positioned between the branch mirror 22 and the imaging element 24. And, the opening formed in the confocal diaphragm is positioned at the fundus conjugate position or in the vicinity of the fundus conjugate position. By providing such a confocal diaphragm, unnecessary light including the reflected light from sites other than the fundus Ef, etc. can be removed. As a result, clearer fundus images can be acquired.

In the ophthalmic apparatus 1 having such a configuration, light generated by the light source 11 uniformly illuminates the slit diaphragm 12. The light that has passed through the slit 12A formed in the slit diaphragm 12 transmits the relay lens 13 as the slit-shaped illumination light, passes through the illumination openings 21Ba and 21Bb formed in the dividing filter 21, and is deflected by the scanning mirror 14. The illumination light deflected by the scanning mirror 14 transmits the relay lens 15, transmits the objective lens 16, and is irradiated onto the fundus Ef through the pupil Eu.

The returning light of the illumination light from the fundus Ef is emitted from the eye through the pupil Eu, transmits the objective lens 16, transmits the relay lens 15, and is descanned by the scanning mirror 14. The returning light descanned by the scanning mirror 14 passes through the light receiving opening 21A formed in the dividing filter 21, and is deflected by the branch mirror 22 toward the descanning optical system 20. The returning light deflected by the branch mirror 22 transmits the relay lens 23, and is imaged on the imaging surface of the imaging element 24.

### [Processing system]

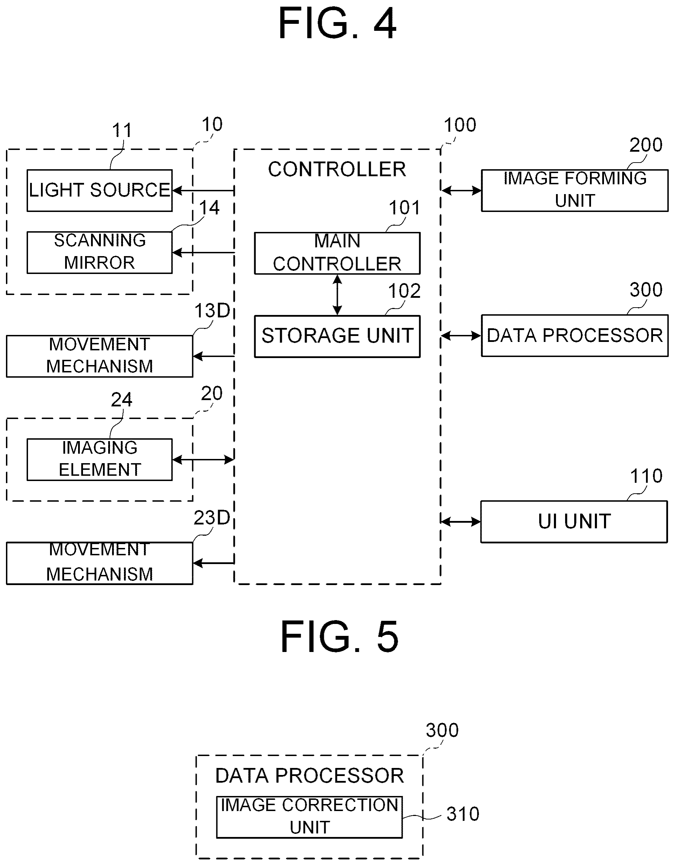

FIG. 4 and FIG. 5 show examples of a configuration of a processing system of the ophthalmic apparatus 1 according to the first embodiment . In FIG. 4, like reference numerals designate like parts as in FIGs. 1 to 3. The same description may not be repeated below. In FIG. 5, parts similar to those in FIG. 4 are denoted by the same reference symbols, and description thereof is omitted as appropriate.

The processing system (control system) of the ophthalmic apparatus 1 is configured with a controller 100 as a center. The controller 100 controls each part of the ophthalmic apparatus 1.

The controller 100 includes a main controller 101 and a storage unit 102. The functions of the main controller 101 are realized by a processor, for example. The storage unit 102 stores, in advance, computer programs for controlling the ophthalmic apparatus 1. The computer programs include program for controlling the scanning optical system, program for controlling the descanning optical system, program for controlling the scanning mirror, program for image forming, program for data processing, program for user interface, and the like. The main controller 101 operates according to such the computer programs, and thereby the controller 100 performs the control processing.

### (Main controller 101)

The main controller 101 controls each of the scanning optical system 10 including the scanning mirror 14, the descanning optical system 20 including the imaging element 24, and movement mechanisms 13D and 23D. Further, the main controller 101 controls an image forming unit 200, a data processor 300, and a user interface (UI) unit 110.

Examples of the control for the scanning optical system 10 include control for the light source 11 and control for the scanning mirror 14.

Examples of the control for the light source 11 include turning the light source on and off, and adjustment of light quantity of the emitted light.

Examples of the control for the scanning mirror 14 include control of the angle of the deflected surface for deflecting the illumination light. By controlling the angle of the deflected surface, the deflection direction (scan direction) of the illumination light can be controlled. By controlling an angular range of the deflected surface, the scan range (scan start position and scan end position) can be controlled. By controlling the speed of changing the angle of deflected surface, the scan speed can be controlled.

Examples of the control for the descanning optical system 20 include control for the imaging element 24.

Examples of the control for the imaging element 24 include control of setting the light receivable region on the imaging surface and control of reading out the light receiving result(s) from the imaging element 24.

The movement mechanism 13D moves the relay lens 13 in the direction of the optical axis O. The main controller 101 outputs control signal(s) to the movement mechanism 13D to move the relay lens 13 in a movement direction corresponding to the control signal(s) by the amount of movement corresponding to the control signal(s). For example, the ophthalmic apparatus 1 is provided with an actuator that generates a driving force for driving the movement mechanism 13D and a transmission mechanism that transmits the driving force from the actuator to the movement mechanism 13D. The actuator includes a pulse motor, for example. The transmission mechanism includes a combination of gears, a rack and pinion, and the like, for example. The movement mechanism 13D receives the driving force generated by the actuator controlled by the main controller 101 from the transmission mechanism, and moves the relay lens 13 in the direction of the optical axis O.

The movement mechanism 23D moves the relay lens 23 in the optical axis direction of the descanning optical system 20. The main controller 101 outputs control signal(s) to the movement mechanism 23D to move the relay lens 23 in a movement direction corresponding to the control signal(s) by the amount of movement corresponding to the control signal(s). For example, the ophthalmic apparatus 1 is provided with an actuator that generates a driving force for driving the movement mechanism 23D and a transmission mechanism that transmits the driving force from the actuator to the movement mechanism 23D. The actuator includes a pulse motor, for example. The transmission mechanism includes a combination of gears, a rack and pinion, and the like, for example. The movement mechanism 23D receives the driving force generated by the actuator controlled by the main controller 101 from the transmission mechanism, and moves the relay lens 23 in the optical axis direction of the descanning optical system 20.

In some embodiments, the ophthalmic apparatus 1 includes an optical system movement mechanism configured to relatively move the optical system relative to the eye E to be examined. The optical system movement mechanism three-dimensionally moves the optical system (optical system of apparatus) shown in FIG. 1. For example, the optical system movement mechanism include a first mechanism, a second mechanism, and a third mechanism. The first mechanism is configured to move the optical system (housing that houses the optical system) in a horizontal direction (left-right direction). The second mechanism is configured to move the optical system in a vertical direction (up-down direction). The third mechanism is configured to move the optical system in the direction of the optical axis O of the scanning optical system 10 (depth direction, front-back direction, working distance direction). For example, the first mechanism includes a first stage movable in the horizontal direction and a first movement mechanism for moving the first stage. For example, the second mechanism includes a second stage movable in the vertical direction and a second movement mechanism for moving the second stage. For example, the third mechanism includes a third stage movable in the direction of the optical axis O and a third movement mechanism for moving the third stage. Each movement mechanism includes a pulse motor as an actuator and operates under the control from the main controller 101.

The control for the optical system movement mechanism is used for alignment and/or tracking. Here, tracking is to move the optical system of apparatus according to the eye movement of the eye to be examined to be photographed. To perform tracking, alignment and focus adjustment are performed in advance. The tracking is a function of maintaining a suitable positional relationship in which alignment and focusing are matched by causing the position of the optical system of the apparatus and the like to follow the eye movement.

In the case of manual alignment, a user operates the UI unit 110 to relatively move the optical system and the eye E to be examined so as to cancel the displacement of the eye to be examined, which is to be photographed, relative to the optical system. For example, the main controller 101 controls the optical system movement mechanism to relatively move the optical system relative to the eye E to be examined, by outputting a control signal corresponding to the operation content for the UI unit 110 to the first movement mechanism, the second movement mechanism, and the third movement mechanism.

In the case of automatic alignment, the main controller 101 controls the optical system movement mechanism to relatively move the optical system relative to the eye E to be examined so as to cancel the displacement of the eye to be examined, which is to be photographed, relative to the optical system. For example, the ophthalmic apparatus 1 includes two or more anterior segment cameras for photographing an anterior segment of the eye E to be examined from different directions. For example, the main controller 101 acquires analysis result(s) of two anterior segment images captured by the two anterior segment cameras. The main controller 101 identifies positional relationship between the two anterior segment cameras and the eye E to be examined based on the acquired analysis result(s), and controls the optical system movement mechanism so that the positional relationship of the optical system relative to the eye E to be examined becomes a predetermined positional relationship based on the identified positional relationship.

Examples of the control for the image forming unit 200 include image forming control for forming images of the eye E to be examined from the light receiving result(s) obtained using the imaging element 24.

Examples of the control for the data processor 300 include image processing control for the images formed by the image forming unit 200.

Examples of the control for the UI unit 110 include control for a display device and control for an operation device (input device).

### (Storage unit 102)

The storage unit 102 stores various types of data. Examples of the data stored in the storage unit 102 include light receiving result(s) obtained by the imaging element 24, image data of an image formed by the image forming unit 200, processing result(s) obtained by the data processor 300, and information on eye to be examined. The information on the eye to be examined includes information on the examinee such as patient ID and name, and information on the eye to be examined such as identification information of the left/right eye.

In addition, the storage unit 102 stores various kinds of computer programs and data for operating the ophthalmic apparatus 1.

### (Image forming unit 200)

The image forming unit 200 forms the light receiving image based on the light receiving result(s) read out from the imaging element 24 under the control from the main controller 101 (controller 100). The image forming unit 200 can sequentially form light receiving images corresponding to the (virtual) light receivable regions on the imaging element 24, and can form a fundus image representing the morphology of the fundus Ef of the eye E to be examined from a plurality of the formed light receiving images. The various images (the various image data) formed by the image forming unit 200 are stored in the storage unit 102, for example.

For example, the image forming unit 200 includes a processor, and executes the function described above by performing processing corresponding to the program(s) stored in the storage unit or the like.

### (Data processor 300)

The data processor 300 executes various kinds of image processing on the light receiving results acquired from the imaging element 24. Examples of the image processing include noise removal processing on the light receiving results, and luminance (brightness) correction processing for easily identifying a predetermined site depicted in the light receiving image based on the light receiving results.

The data processor 300 includes a processor, and realizes the above functions by performing processing corresponding to the program(s) stored in the storage unit or the like.

FIG. 5 shows a block diagram of an example of a configuration of the data processor 300.

The data processor 300 includes an image correction unit 310. The image correction unit 310 executes (performs) black spot shadow correction processing on the fundus image of the eye E to be examined formed by the image forming unit 200. In the acquired fundus image of the eye E to be examined, two black spot shadows, each of which corresponds to each of the illumination openings 21Ba and 21Bb, are depicted. The black spot shadow correction processing is executed on at least one of a plurality of black spot shadows depicted in the fundus image of the eye E to be examined.

The black spot shadow correction processing is processing of correcting the gradation information (luminance information, luminance distribution) of a region of the black spot shadow depicted in the image to make the black spot shadow less noticeable relative to the surrounding region or to eliminate the black spot shadow itself.

The first example of the black spot shadow correction processing is processing of correcting the gradation value(s) (luminance value(s), luminance distribution) within the region of the black spot shadow based on the gradation (brightness) surrounding the region of the black spot shadow depicted in the fundus image. For example, the gradation value within the region of the black spot shadow is corrected based on a statistical value of the luminance distribution of the region surrounding the region of the black spot shadow. Examples of the statistical value include a median value, an average value, a maximum value, a minimum value, and a mode value.

In the second example of the black spot shadow correction processing, a region to be corrected and correction amount are identified based on the region of the black spot shadow identified in a simulation image obtained in advance by executing an optical simulation using an optical condition of the ophthalmic apparatus 1. For example, the image correction unit 310 corrects the region of the black spot shadow by changing luminance in a predetermined region in the fundus image, which is acquired by the image forming unit 200, by a predetermined amount, using the region to be corrected and the correction amount that are identified in advance.

In some embodiments, the image correction unit 310 identifies the region of the black spot shadow in the fundus image by analyzing the fundus image of the eye E to be examined. For example, the region of the black spot shadow is identified based on changes in gradation values of a contour of a region of the black spot shadow or a known shape of the region of the black spot shadow. The image correction unit 310 can executes the black spot shadow correction processing described above on the identified region.

The scanning optical system 10 is an example of the "first scanning optical system" or the "illumination optical system" according to the embodiments. The descanning optical system 20 is an example of the "light receiving optical system" or the "photographing (imaging) optical system" according to the embodiments. The light source 11 and the slit diaphragm 12 are an example of the "light source unit" according to the embodiments. The branch mirror 22 is an example of the "deflecting element" according to the embodiments.

### <Operation example>

Next, an example of an operation of the ophthalmic apparatus 1 according to the first embodiment will be described.

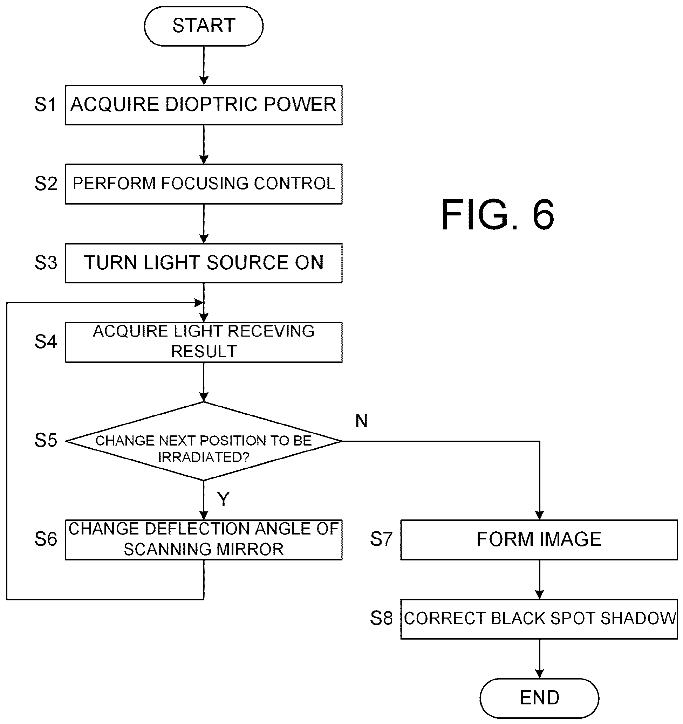

FIG. 6 shows an example of an operation of the ophthalmic apparatus 1 according to the first embodiment. FIG. 6 shows a flowchart of the example of the operation of the ophthalmic apparatus 1 according to the first embodiment. The storage unit 102 stores a of computer programs for realizing the processing shown in FIG. 6. The main controller 101 operates according to the computer program, and thereby the main controller 101 performs the processing shown in FIG. 6.

It is assumed that, prior to the processing shown in FIG. 6, the alignment of the optical system relative to the eye E to be examined has been completed, and the deflected surface of the scanning mirror 14 has been set in a predetermined deflection start angle direction.

### (S1: Acquire dioptric power)

First, the main controller 101 acquires the dioptric power (refractive power) of the eye E to be examined. For example, the main controller 101 acquires the dioptric power of the eye E to be examined from an external ophthalmic measurement apparatus or an electronic medical record. Alternatively, the main controller 101 acquires the dioptric power designated by the user using the UI unit 110.

### (S2: Perform focusing control)

Next, the main controller 101 controls the movement mechanisms 13D and 23D based on the dioptric power of the eye E to be examined acquired in step S1. Specifically, the main controller 101 identifies a position of the relay lens 13 on the optical axis O and a position of the relay lens 23 on the optical axis of the descanning optical system 20, that correspond to the dioptric power of the eye E to be examined. The main controller 101 controls the movement mechanisms 13D and 23D so that the relay lenses 13 and 23 move to the identified positions, respectively.

In some embodiment, the main controller 101 moves the relay lens 13 in the direction of the optical axis O by a movement amount in a movement direction corresponding to the dioptric power of the eye E to be examined. And, the main controller 101 also moves the relay lens 23 in the optical axis direction of the descanning optical system 20 by a movement amount in a movement direction corresponding to the dioptric power of the eye E to be examined.

In some embodiments, the movement mechanisms 13D and 23D are configured to move the relay lenses 13 and 23 in conjunction with each other.

### (S3: Turn light source on)

Subsequently, the main controller 101 sets the light source 11 to on, and turns the light source 11 on.

Thereby, as described above, the scanning optical system 10 starts scanning a predetermined photographing region on the fundus Ef using the slit-shaped illumination light.

### (S4: Acquire light receiving result)

The main controller 101 controls the imaging element 24 to acquire light receiving results of the pixels in the light receivable region on the imaging surface of the imaging element 24 that receives the returning light of the illumination light from the irradiated region of the fundus Ef.

### (S5: Change next position to be irradiated?)

The main controller 101 determines whether or not the irradiated position to be irradiated next with the illumination light is present. The main controller 101 can determine whether or not the next irradiated position is to be irradiated with the illumination light, by determining whether or not the irradiated range of the illumination light that is moved sequentially has covered a predetermined imaging range of the fundus Ef.

When it is determined that the irradiated position to be irradiated next with the illumination light is present (S5: Y), the operation of the ophthalmic apparatus 1 proceeds to step S6. When it is determined that the irradiated position to be irradiated next with the illumination light is not present (S5: N), the operation of the ophthalmic apparatus 1 proceeds to step S7.

### (S6: Change deflection angle of scanning mirror)

When it is determined in step S5 that the irradiated position to be irradiated next with the illumination light is present (S5: Y), the main controller 101 controls the scanning mirror 14 to change the deflected surface of the scanning mirror 14 by a predetermined angle.

Subsequent to step S6, the processing of the ophthalmic apparatus 1 proceeds to step S4.

### (S7: Form image)

In step S5, when it is determined that the next irradiated position is not to be irradiated with the illumination light (S5: N), the main controller 101 controls the image forming unit 200 to form the fundus image of the eye E to be examined from the light receiving results acquired repeatedly while changing an irradiated range of the illumination light in steps S4 to S6.

For example, the image forming unit 200 syntheses a plurality of light receiving results with different irradiated ranges of the illumination light for the number of times repeating the process in steps S4 to S6, based on the order of the movement of the irradiated range. Thereby, the fundus image for one frame of the fundus Ef of the eye E to be examined is formed.

In some embodiments, in step S6, the illumination light is irradiated on the irradiated range set so as to have an overlapping region with the adjacent irradiated range. Thereby in step S7, the fundus image for one frame is formed by synthesizing the light receiving results so that the overlap regions overlap with each other.

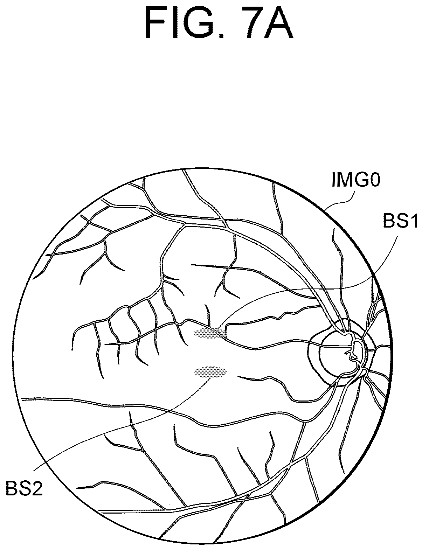

FIG. 7A schematically shows the fundus image formed in step S7. It is assumed that, in FIG. 7A, the fundus image IMG0 formed in step S7 is clipped into a circular shape similar to that of a fundus image acquired by the conventional ophthalmic apparatus.

In the fundus image IMG0, the black spot shadows BS1 and BS2 are depicted corresponding to the illumination openings 21Ba and 21Bb, respectively. Here, it is assumed that the black spot shadow BS1 corresponds to the illumination opening 21Ba, and the black spot shadow BS2 corresponds to the illumination opening 21Bb.

In a region of the black spot shadow BS1 formed corresponding to the illumination opening 21Ba, the fundus information (gradation information) is included caused by the light quantity of the returning light of the illumination light that has passed through the illumination opening 21Bb other than the illumination opening 21Ba. In a region of the black spot shadow BS2 formed corresponding to the illumination opening 21Bb, the fundus information (gradation information) is included caused by the light quantity of the returning light of the illumination light that has passed through the illumination opening 21Ba other than the illumination opening 21Bb.

### (S8: Correct black spot shadow)

Next, the main controller 101 controls the image correction unit 310 to execute the black spot shadow correction processing on the fundus image formed in step S7. The image correction unit 310 corrects the black spot shadow by changing luminance in the regions of the black spot shadow BS1 and BS2 in the fundus image by predetermined amounts from the regions to be corrected and the correction amounts that are identified based on the simulation result executed in advance.

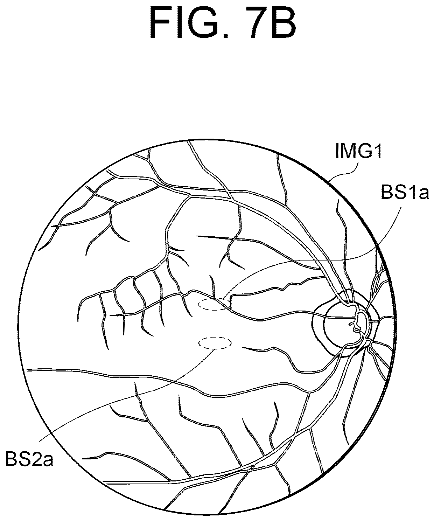

FIG. 7B schematically shows the fundus image corrected in step S8. In FIG. 7B, the fundus image on which the black spot shadow correction processing is performed on the fundus image IMG0 shown in FIG. 7A is illustrated.

The image correction unit 310 generates the fundus image IMG1 including the regions BS1a and BS2a in which the luminance of the regions of the black spot shadows BS1 and BS2 have been corrected, by performing the black spot shadow correction processing on the fundus image IMG0, as described above.

This terminates the operation of the ophthalmic apparatus 1 (END).

In FIG. 6, steps S1 to S3, which are not technically related to steps S4 to S6, may not necessarily be executed. In the same way, steps S4 to S6 are not technically related to steps S7 and S8. Therefore, even when steps S7 and S8 are executed, steps S4 to S6 may not be executed. Even when steps S4 to S6 are executed, steps S7 and S8 may not be executed.

As explained above, according to the first embodiment, the fundus image is acquired by illuminating the fundus Ef of the eye E to be examined with the illumination light that has passed through the two illumination openings 21Ba and 21Bb. In the fundus image, the two black spot shadows BS1 and BS2 corresponding to the illumination openings 21Ba and 21Bb, respectively, are depicted. Each black spot shadow includes the gradation information obtained by illuminating with the illumination light that has passed through the remaining illumination openings excluding the corresponding illumination opening. As a result, each of the two or more black spot shadows BS1 and BS2 is not in a state of blackout, and a region of each black spot shadow includes the gradation information (intraocular information, fundus information) corresponding to the light quantity of the illumination light that has passed through the remaining illumination openings or the light quantity of the returning light that has passed through the remaining light receiving openings. Thereby, by performing the black spot correction processing on the fundus image in which the black spot shadow(s) as described above are depicted, the fundus image useful for diagnosis can be acquired without losing fundus information.

Therefore, even when the small pupil photography is performed by shortening the gap that is a distance between the illumination opening and the light receiving opening on the pupil conjugate plane, the fundus image useful for diagnosis of the eye with microcoria can be acquired.

### <Second embodiment>

The configuration of the ophthalmic apparatus according to the embodiments is not limited to the configuration of the ophthalmic apparatus 1 according to the first embodiment. For example, in the ophthalmic apparatus according to the embodiments, another scanning optical system (second scanning optical system) different from the scanning optical system 10 may be arranged between the dividing filter 21 (branch mirror 22) and the imaging element. In this case, the another scanning optical system different from the scanning optical system 10 is configured to deflect the descanned returning light of the illumination light to move the light-receptive region of the returning light on the imaging surface of the imaging element (i.e., to scan again). Thereby, without being affected by the deflection operation characteristics of the scanning mirror 14, the fundus image of the eye E to be examined can be acquired.

In the following, the ophthalmic apparatus according to a second embodiment will be described focusing on differences from the ophthalmic apparatus 1 according to the first embodiment.

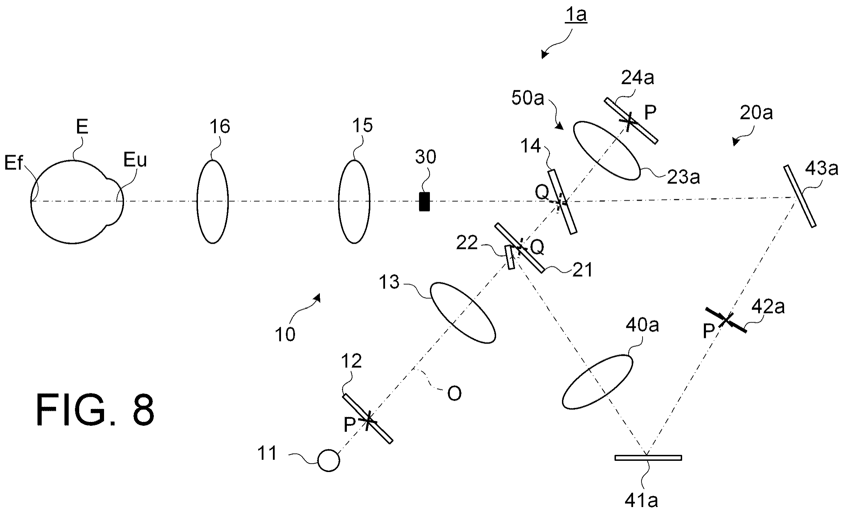

FIG. 8 shows an example of a configuration of the optical system of the ophthalmic apparatus according to the second embodiment. In FIG. 8, like parts are designated by like reference numerals as in FIG. 1 and repetitious description of such parts may not be provided.

The first point of differences between the configuration of the optical system of an ophthalmic apparatus 1a according to the second embodiment and the configuration of the optical system of the ophthalmic apparatus 1 according to the first embodiment is that an imaging element 24a is positioned instead of the imaging element 24. The second point of the differences is that a descanning optical system 20a is positioned instead of the descanning optical system 20. The third point of the differences is that a scanning optical system 50a is positioned between the imaging element 24a and the descanning optical system 20a.

### (Descanning optical system 20a)

The descanning optical system 20a includes a relay lens 40a, a deflecting mirror 41a, a confocal diaphragm 42a, and a deflecting mirror 43a.

The relay lens 40a transmits the descanned returning light of the illumination light that has been deflected by the branch mirror 22 to guide the returning light to the deflecting mirror 41a. The returning light that has been guided to the deflecting mirror 41a is deflected toward the confocal diaphragm 42a.

The confocal diaphragm 42a is positioned at the fundus conjugate position or in the vicinity of the fundus conjugate position. Specifically, an opening formed in the confocal diaphragm 42a is positioned at the fundus conjugate position or in the vicinity of the fundus conjugate position. Thereby, unnecessary light can be eliminated in a state where an optical path of the returning light is fixed, and clearer fundus images can be acquired. The retuning light that has passed through the opening formed in the confocal diaphragm 42a is guided to the deflecting mirror 43a.

### (Scanning optical system 50a)

In FIG. 8, the scanning operation of the returning light in the scanning optical system 50a is performed by the scanning mirror 14 that realizes the scanning operation of the illumination light in the scanning optical system 10.

For example, deflected surfaces (reflective surfaces) are provided on both surfaces of the scanning mirror 14. The scanning mirror 14 deflects the illumination light and the returning light of the illumination light that has been guided through the objective lens 16, on a first deflected surface which is a front surface. Further, the scanning mirror 14 deflects the returning light (returning light that has passed through the light receiving opening formed in the dividing filter 21) deflected by the deflecting mirror 43a on a second deflected surface which is a bask surface of the first deflected surface.

In some embodiments, the scanning operation of the returning light in the scanning optical system 50a is performed by another scanning mirror (optical scanner) different from the scanning mirror 14. In this case, the deflection operation characteristics of the another scanning mirror are substantially the same as those of the scanning mirror 14, and the deflection operation of the another scanning mirror is configured to be synchronized with that of the scanning mirror 14.

The scanning optical system 50a includes a relay lens 23a and the imaging element 24a. The scanning optical system 50a may include the scanning mirror 14 that deflects the returning light on the second deflected surface.

### (Relay lens 23a)

The relay lens 23 transmits the returning light deflected on the second deflected surface of the scanning mirror 14 to guide the returning light to the imaging element 24a (imaging surface).

In some embodiment, the relay lens 23a is configured to be capable of moving in an optical axis direction of the scanning optical system 50a, in the same way as the relay lens 23, as a focusing lens. By moving the relay lens 23a in the optical axis direction of the scanning optical system 50a, the imaging element 24a (imaging surface) can be arranged at the fundus conjugate position or in the vicinity of the fundus conjugate position.

In some embodiment, the imaging element 24a is configured to be capable of moving in the optical axis direction of the scanning optical system 50a.

### (Imaging element 24a)

The imaging element 24a (imaging surface, light receiving surface, detection surface) can be arranged at the fundus conjugate position or in the vicinity of the fundus conjugate position. The imaging element 24a realizes the function of a two-dimensional image sensor as a pixelated photodetector. Specifically, the imaging surface (detection surface, light receiving surface) of the imaging element 24a can be arranged at the fundus conjugate position or in the vicinity of the fundus conjugate position. The imaging element 24a is configured to be capable of setting a light receivable region (light-receptive region) that can be virtually moved at the fundus conjugate position or in the vicinity of the fundus conjugate position.

For example, the light receiving result(s) acquired by the imaging element 24a is/are captured and read out using a rolling shutter method (system). In some embodiments, the light receiving result(s) acquired by the imaging element 24a is/are captured and read out using a global shutter system in which the light receivable region can be changed or be moved. In some embodiments, a controller according to the second embodiment performs readout control of the light receiving result(s) by controlling the imaging element 24a. In some embodiments, the imaging element 24a can automatically output the light receiving result(s) for a predetermined number of lines, along with information indicating the light receiving position(s).

The imaging element 24a with this configuration includes a CMOS image sensor. In some embodiments, the imaging element 24a includes a CCD image sensor, for example.

By capturing (reading out) the light receiving result(s) of the returning light using the rolling shutter method for the imaging element 24a, the image in the light receivable region corresponding to a desired virtual opening shape extending in a predetermined direction is acquired. The image forming unit 200 forms the fundus image of the eye E to be examined based on light receiving result(s) of the imaging element 24a read out using rolling shutter method.

In the ophthalmic apparatus 1a having such a configuration, light generated by the light source 11 uniformly illuminates the slit diaphragm 12. The light that has passed through the slit 12A formed in the slit diaphragm 12 transmits the relay lens 13 as the slit-shaped illumination light, passes through the illumination openings 21Ba and 21Bb formed in the dividing filter 21, and is deflected on the first deflected surface of the scanning mirror 14. The illumination light deflected on the first deflected surface of the scanning mirror 14 transmits the relay lens 15, transmits the objective lens 16, and is irradiated onto the fundus Ef through the pupil Eu.

The returning light of the illumination light from the fundus Ef is emitted from the eye through the pupil Eu, transmits the objective lens 16, transmits the relay lens 15, and is descanned on the first deflected surface of the scanning mirror 14. The returning light descanned on the first deflected surface of the scanning mirror 14 passes through the light receiving opening 21A formed in the dividing filter 21, and is deflected by the branch mirror 22 toward the descanning optical system 20a. The returning light deflected by the branch mirror 22 transmits the relay lens 40a, is deflected by the deflecting mirror 41a, passes through the opening formed in the confocal diaphragm 42a, and is deflected by the deflecting mirror 43a toward the second deflected surface of the scanning mirror 14. The returning light deflected by the deflecting mirror 43a is deflected on the second deflected surface of the scanning mirror 14, transmits the relay lens 23a, and is imaged on the imaging surface of the imaging element 24a.

A configuration of a processing system of the ophthalmic apparatus 1a according to the second embodiment is the same configuration as that of the ophthalmic apparatus 1 according to the first embodiment.

The first point of differences between the control of the controller 100 (main controller 101) according to the second embodiment and that of the controller 100 according to the first embodiment is that a target to be controlled of the movement mechanism 23D is changed from the relay lens 23 to the relay lens 23a. The second point of the differences is that the controller 100 controls the imaging element 24a instead of the imaging element 24.

The control for the imaging element 24a includes control of setting the light receivable region on the imaging surface, and control for reading out the light receiving result(s) using the rolling shutter method (for example, setting of light receiving size corresponding to the size of the illumination pattern, or the like). In addition, examples of the control for the imaging element 24a include the reset control, the exposure control, the charge transfer control, and the output control.

The operation of the ophthalmic apparatus 1a according to the second embodiment is similar to the operation of the ophthalmic apparatus 1 according to the first embodiment. Thus, the detailed description of the operation will be omitted.

As explained above, according to the second embodiment, in the same way as the first embodiment, the two black spot shadows BS1 and BS2 corresponding to the illumination openings 21Ba and 21Bb, respectively, are depicted in the fundus image. Each black spot shadow includes the gradation information obtained by illuminating with the illumination light that has passed through the remaining illumination openings excluding the corresponding illumination opening. As a result, each of the two or more black spot shadows BS1 and BS2 is not in a state of blackout, and a region of each black spot shadow includes the gradation information (intraocular information, fundus information) corresponding to the light quantity of the illumination light that has passed through the remaining illumination openings or the light quantity of the returning light that has passed through the remaining light receiving openings. Thereby, by performing the black spot correction processing on the fundus image in which the black spot shadow(s) as described above is/are depicted, the fundus image useful for diagnosis can be acquired without losing fundus information.

Therefore, according to the second embodiment, in the same way as the first embodiment, even when the small pupil photography is performed by shortening the gap that is a distance between the illumination opening and the light receiving opening on the pupil conjugate plane, the fundus image useful for diagnosis of the eye with microcoria can be acquired.

### <Modification example>

The configuration of the ophthalmic apparatus according to the embodiments is not limited to the configurations of the ophthalmic apparatus according to the first embodiment or the second embodiment.

### [First modification example]

As describe above, the shape of the illumination opening 21Ba or 21Bb formed in the dividing filter 21 according to the embodiments is not limited to the rectangle described in the first embodiment or the second embodiment. The shape of the illumination opening 21Ba or 21Bb may be any shape.

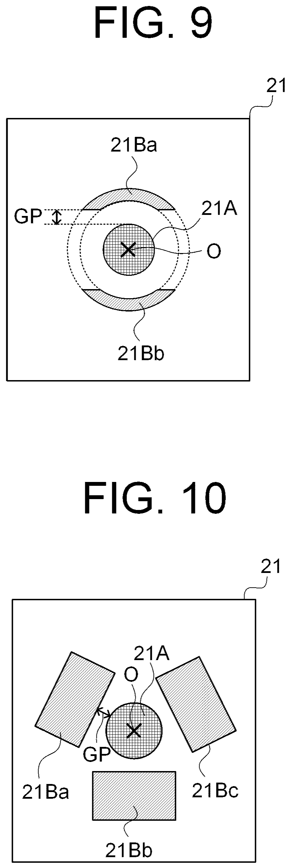

FIG. 9 shows an explanatory diagram of the dividing filter 21 according to a first modification example of the first embodiment or the second embodiment. FIG. 9 schematically represents an example of a configuration of the dividing filter 21 when viewed from the optical axis O of the scanning optical system 10, in the same way as in FIG. 3.

Each of the illumination openings 21Ba and 21Bb formed in the dividing filter 21 according to the present modification example is a part of a ring-shaped opening centered at a position corresponding to the optical axis O. That is, each of the illumination openings 21Ba and 21Bb is a part of a ring-shaped opening having a predetermined width in a radial direction centered at a position corresponding to the optical axis O. Further, the illumination openings 21Ba and 21Bb are formed at positions that are point-symmetrical with reference to a position corresponding to the optical axis O.

Furthermore, in the dividing filter 21 according to the present modification example, the light receiving opening 21A is formed in a central region including a position corresponding to the optical axis O. Both ends in a circumferential direction of each of the illumination openings 21Ba and 21Bb have shapes of notching so that the gap GP is a predetermined gap length.

In some embodiments, at least one of the illumination openings 21Ba or 21Bb is formed into a circular arc shape, an arc shape, an arcuate shape (shape formed by a portion of a circular arc and a chord connecting both ends of the circular arc), or a polygonal shape formed by three or more line segments.

### [Second modification example]

As describe above, the illumination openings formed in the dividing filter 21 according to the embodiments are limited to the illumination openings 21Ba and 21Bb as explained in the first embodiment or the second embodiment. The illumination openings may be three or more.

FIG. 10 shows an explanatory diagram of the dividing filter 21 according to a second modification example of the first embodiment or the second embodiment. FIG. 10 schematically represents an example of a configuration of the dividing filter 21 when viewed from the optical axis O of the scanning optical system 10, in the same way as in FIG. 3.

In the dividing filter 21 according to the present modification example, a single light receiving opening is formed in a central region including a position corresponding to the optical axis O, and three illumination openings 21Ba, 21Bb, and 21Bc are formed in a peripheral region eccentric to the optical axis O.

Each of the illumination openings 21Ba, 21Bb, and 21Bc is formed at a distance separated by a distance of the gap GP from the light receiving opening 21A.

In the present modification example, at least one of the shapes of the illumination openings 21Ba, 21Bb, or 21Bc may be the same as the shapes described in the first modification example.

### [Third modification example]

In the first embodiment or the second embodiment, the case where the two or more illumination openings and the single light receiving opening are formed in the dividing filter 21 has been described. However, the configuration of the dividing filter 21 according to the embodiments is not limited thereto. In the dividing filter 21 according to the embodiments, a single illumination opening may be formed in a central region including a position corresponding to the optical axis O, and two or more light receiving openings may be formed in a peripheral region eccentric to the optical axis O.

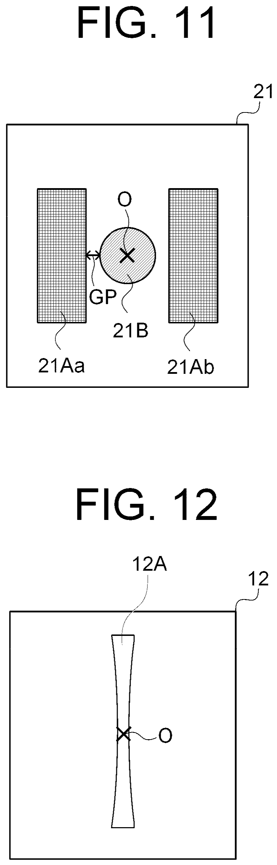

FIG. 11 shows an explanatory diagram of the dividing filter 21 according to a third modification example of the first embodiment or the second embodiment. FIG. 11 schematically represents an example of a configuration of the dividing filter 21 when viewed from the optical axis O of the scanning optical system 10, in the same way as in FIG. 3.

It is assumed that, in the dividing filter 21 according to the present modification example, a single illumination opening 21B is formed in a central region including a position corresponding to the optical axis O, and two light receiving openings 21Aa and 21Ab are formed in a peripheral region eccentric to the optical axis O.

The illumination opening 21B is formed in the central region including the position corresponding to the optical axis O as an optical axis of the illumination light. The two light receiving openings 21Aa and 21Ab are formed in the peripheral region eccentric to the optical axis O. The illumination opening 21B and the light receiving opening 21Aa (21Ab) are formed separated by a distance of the gap GP.

In FIG. 11, the shape of the illumination opening 21B is a circular shape or a shape of an ellipse. However, the configuration according to the embodiments is not limited to the shape of the illumination opening 21B. In addition, the shapes of the light receiving openings 21Aa and 21Ab are rectangles (rectangular shapes, square shapes). However, the configuration according to the embodiments is not limited to the shape of the light receiving opening 21Aa or 21Ab. Further, the shapes of the two or more light receiving openings including the light receiving openings 21Aa and 21Ab may be different from each other.

In the present modification example, each of the one or more black spot plates 30 is positioned at or in the vicinity of a position where the illumination light reflected by the objective lens 16 is imaged in the scanning optical system 10 with the single illumination opening 21B as an object point.

The scanning optical system 10 is configured to irradiate the slit-shaped illumination light onto the dividing filter 21 so as to pass through the illumination opening 21B formed in the dividing filter 21. The descanning optical system 20 (20a) is configured so that the returning light of the illumination light that has passed through the light receiving openings 21Aa and 21Ab formed in the dividing filter 21 is guided by the branch mirror 22, etc.

### [Fourth modification example]

In the first embodiment or the second embodiment, the shape of slit 12A formed in slit diaphragm 12 is described as rectangular. However, the configuration of the embodiments is not limited to this.

FIG. 12 shows an explanatory diagram of the slit diaphragm 12 according to the first embodiment or the second embodiment. FIG. 12 schematically represents an example of a configuration of the slit diaphragm 12 when viewed from the optical axis O of the scanning optical system 10, in the same way as in FIG. 2.

The slit 12A formed in the slit diaphragm 12 according to the present modification example has a shape in which the width in the shorter direction (perpendicular to the longitudinal direction) passing through the optical axis O is shorter than the width in the shorter direction at the end in the slit direction. For example, the width in the shorter direction of the slit 12A is formed so as to be the same or increase toward the end in the slit direction from the optical axis O.

According to the present modification example, the light quantity of the illumination light passing through the end of the slit 12A, which is away from the optical axis O, and irradiating onto the fundus Ef can be increased. Thereby, the illumination unevenness in the irradiated region of the illumination light irradiated onto the fundus Ef can be reduced.

The configuration according to any one of the first modification example to the fourth modification example describe above can be applied to the configurations of the remaining modification examples of the first modification example to the fourth modification example.

### [Actions]

The ophthalmic apparatus according to the embodiments will be described.

The first aspect of the embodiments is an ophthalmic apparatus (1, 1a) including a first scanning optical system (scanning optical system 10), a dividing filter (21), a descanning optical system (20), and one or more black spot plates (30). The first scanning optical system includes an objective lens (16) and a scanning mirror (14) positioned at a position substantially conjugate optically to a pupil (Eu) of an eye (E) to be examined. The first scanning optical system is configured to deflect slit-shaped illumination light from a light source unit using the scanning mirror to guide the deflected illumination light to the eye to be examined through the objective lens. The dividing filter is positioned at a position substantially conjugate optically to the pupil between the light source unit and the scanning mirror. In the dividing filter, two or more illumination openings (21Ba, 21Bb) and a single light receiving opening (21A), or a single illumination opening (21B) and two or more light receiving openings (21Aa, 21Ab) are formed. The descanning optical system is configured to guide returning light of the illumination light from the eye to be examined to an imaging element (24, 24a). Here, the returning light is guided through the objective lens and the scanning mirror and passes through the light receiving opening. The imaging element is positioned at a position substantially conjugate optically to a photographed site of the eye to be examined. The one or more black spot plates are positioned at positions where the illumination light reflected by the objective lens is imaged in the first scanning optical system on an optical path between the objective lens and the scanning mirror.

According to such an aspect, in an image of a photographed site of the eye to be examined acquired by the imaging element, two or more black spot shadows are depicted corresponding to the two or more illumination openings or the two or more light receiving openings. Each black spot shadow includes intraocular information (gradation information) obtained by illuminating with the illumination light that has passed through the remaining illumination openings excluding the corresponding illumination opening(s) among the two or more illumination openings. Or, each black spot shadow includes intraocular information obtained by the returning light that has passed through the remaining light receiving openings excluding the corresponding light receiving opening(s) among the light receiving openings. As a result, each of the two or more black spot shadows is not in a state of blackout, and a region of each black spot shadow includes intraocular information corresponding to the light quantity of the illumination light that has passed through the remaining illumination openings or the light quantity of the returning light that has passed through the remaining light receiving openings.

Therefore, even when the eye to be examined is an eye with microcoria, which requires that the gap between the illumination opening and the light receiving opening be shortened, it becomes possible to acquire images including the intraocular information of the eye to be examined without completely losing the intraocular information, and appropriate diagnoses can be performed on images of the eyes with microcoria.

In the second aspect of the embodiments, in the first aspect, the one or more black spot plates are positioned at positions where the illumination light reflected by the objective lens is imaged in the first scanning optical system with the two or more illumination openings or the single illumination opening as object point.

According to such an aspect, the image including the intraocular information of the eye to be examined can be acquired without completely losing the intraocular information, while efficiently removing artifact(s) alone caused by the illumination light divided by pupil-division using the dividing filter.

In the third aspect of the embodiments, in the first aspect, the light source unit includes: a light source (11); and a slit diaphragm (12) in which a slit-shaped opening (slit 12A) capable of being positioned at a position substantially conjugate optically to the photographed site is formed. The light source unit is configured to emit slit-shaped illumination light by irradiating light from the light source onto the slit diaphragm.

According to such an aspect, the ophthalmic apparatus, which can illuminate inside of the eye of the eye to be examined with the slit-shaped illumination light generated with a simple configuration using the slit diaphragm, and can acquire images including the intraocular information of the eye to be examined without completely losing the intraocular information, can be provided.

In the fourth aspect of the embodiments, in the first aspect, in the dividing filter, the single light receiving opening (21A) is formed in a central region including a position corresponding to an optical axis (O) of the illumination light, and the two or more illumination openings (21Ba, 21Bb) are formed in a peripheral region eccentric to the optical axis.

According to such an aspect, the image of the eye to be examined in which the two or more black spot shadows are depicted can be acquired with a simple configuration, without completely losing the intraocular information by completely dividing the illumination light flux and the light receiving light flux on the pupil conjugate plane.

In the fifth aspect of the embodiments, in the first aspect, in the dividing filter, the single illumination opening (21B) is formed in a central region including a position corresponding to an optical axis (O) of the illumination light, and the two or more light receiving openings (21Aa, 21Ab) are formed in a peripheral region eccentric to the optical axis.

According to such an aspect, the image of the eye to be examined in which the two or more black spot shadows are depicted can be acquired with a simple configuration, without completely losing the intraocular information by completely dividing the illumination light flux and the light receiving light flux on the pupil conjugate plane.

In the sixth aspect of the embodiments, in the first aspect, the photographed site is a fundus (Ef).

According to such an aspect, even when the eye to be examined is an eye with microcoria, which requires that the gap between the illumination opening and the light receiving opening be shortened, fundus images of the eye to be examined can be acquired without completely losing fundus information as the intraocular information. Therefore, appropriate diagnosis can be performed on the image of the eye with microcoria.

In the seventh aspect of the embodiments, in any one of the first embodiment to the sixth aspect, the one or more black spot plates are positioned at at least one of a position where the illumination light reflected on an anterior surface of the objective lens is imaged in the first scanning optical system, or a position where the illumination light reflected on a posterior surface of the objective lens is imaged in the first scanning optical system.

According to such an aspect, the image including the intraocular information of the eye to be examined can be acquired without completely losing the intraocular information, while efficiently removing artifact(s) caused by at least one of the anterior surface of the objective lens or the posterior surface of the objective lens.

In the eighth aspect of the embodiments, in any one of the first aspect to the sixth aspect, the objective lens includes one or more doublets configured by sticking two or more lenses. The one or more black spot plates are positioned at positions where the illumination light reflected on one or more sticking surfaces of the one or more doublets is imaged in the first scanning optical system.

According to such an aspect, the image including the intraocular information of the eye to be examined can be acquired without completely losing the intraocular information, while efficiently removing artifact(s) caused by the one or more sticking surfaces of the objective lens.

The ninth aspect of the embodiments, in any one of the first aspect to the sixth aspect, further includes a deflecting element (branch mirror 22) positioned at a position substantially conjugate optically to the pupil, and configured to deflect the returning light passing through the light receiving opening toward the descanning optical system.

According to such an aspect, the returning light that has passed through the two or more light receiving openings or the single light receiving opening is deflected toward the descanning optical system. Thereby, the returning light can be received with sufficient light quantity, while preventing loss of light quantity of the returning light.

In the tenth aspect of the embodiments, in any one of the first aspect to the sixth aspect, the descanning optical system includes a confocal diaphragm (42a) positioned at a position substantially conjugate optically to the photographed site.

According to such an aspect, unnecessary light from the photographed site, etc. can be eliminated in the descanning optical system. Thereby, clearer images of the eye to be examined can be acquired.

The eleventh aspect of the embodiments, in any one of the first aspect to the sixth aspect, further includes a second scanning optical system (scanning optical system 50a) positioned between the dividing filter and the imaging element, and configured to deflect the returning light to move a light-receptive region on an imaging surface of the imaging element.

According to such an aspect, the light-receptive region of the returning light on the imaging surface of the imaging element is moved by deflecting the returning light through the descanning optical system. Thereby, the light receiving results of the returning light can be acquired without being affected by the deflection operation characteristics of the scanning mirror.

In the twelfth aspect of the embodiments, in the eleventh aspect, the scanning mirror is configured to deflect the illumination light and returning light of the illumination light that is guided through the objective lens on a first deflected surface. The second scanning optical system is configured to deflect the returning light passing through the light receiving opening on a second deflected surface that is backside of the first deflected surface of the scanning mirror.

According to such an aspect, the light receiving results of the returning light can be acquired with a simple configuration, without being affected by the deflection operation characteristics of the scanning mirror.

The thirteenth aspect of the embodiments, in the eleventh aspect, further includes an image forming unit (200) configured to form an image of the eye to be examined based on light receiving result of the imaging element read out using rolling shutter method.

According to such an aspect, without being affected by the deflection operation characteristics of the scanning mirror, clear fundus images of the eye to be examined can be acquired while removing unnecessary light.

The fourteenth aspect of the embodiments, in any one of the first aspect to the sixth aspect, further includes an image correction unit (310) configured to correct a black spot shadow depicted in the image, by performing luminance correction on the image of the eye to be examined acquired based on the light receiving result of the imaging element.

According to such an aspect, a situation in which the intraocular information is completely lost can be avoided by correcting the two or more black spot shadows depicted in the image of the eye to be examined. As a result, even when the eye to be examined is an eye with microcoria, appropriate diagnosis can be performed on the image of the eye with microcoria

In the fifteenth aspect of the embodiments, in the fourteenth aspect, the image correction unit is configured to perform the luminance correction on at least one of two or more black spot shadows depicted in the image.

According to such an aspect, a situation in which the intraocular information is completely lost can be avoided. As a result, even when the eye to be examined is an eye with microcoria, appropriate diagnosis can be performed on the image of the eye with microcoria

In the sixteenth aspect of the embodiments, in the fifteenth aspect, the image correction unit is configured to correct the black spot shadow by changing luminance in a predetermined region in the image by a predetermined amount.

According to such an aspect, the black spot shadow(s) depicted in the image of the eye to be examined can be corrected with a simple processing, using simulation result, etc. executed in advance.

### EXPLANATION OF SYMBOLS

- 1, 1a: Ophthalmic apparatus
- 10, 50a: Scanning optical system
- 11: Light source
- 12: Slit diaphragm
- 13, 15, 23, 23a, 40a: Relay lens
- 14: Scanning mirror
- 16: Objective lens
- 20, 20a: Descanning optical system
- 21: Dividing filter
- 22: Branch mirror
- 24, 24a: Imaging element
- 30: Black spot plate
- 41a, 43a: Deflecting mirror
- 42a: Confocal diaphragm
- 100: Controller
- 101: Main controller
- 102: Storage unit
- 200: Image forming unit
- 300: Data processor
- 310: Image correction unit
- E: Eye to be examined
- Ef: Fundus
- Eu: Pupil
- P: Fundus conjugate position
- Q: Pupil conjugate position

## Claims

1. An ophthalmic apparatus, comprising:
a first scanning optical system including an objective lens and a scanning mirror positioned at a position substantially conjugate optically to a pupil of an eye to be examined, and configured to deflect slit-shaped illumination light from a light source unit using the scanning mirror to guide the deflected illumination light to the eye to be examined through the objective lens;
a dividing filter positioned at a position substantially conjugate optically to the pupil between the light source unit and the scanning mirror, and having formed two or more illumination openings and a single light receiving opening, or a single illumination opening and two or more light receiving openings;
a descanning optical system configured to guide returning light of the illumination light from the eye to be examined to an imaging element, the returning light being guided through the objective lens and the scanning mirror and passing through the light receiving opening, the imaging element being positioned at a position substantially conjugate optically to a photographed site of the eye to be examined; and
one or more black spot plates positioned at positions where the illumination light reflected by the objective lens is imaged in the first scanning optical system on an optical path between the objective lens and the scanning mirror.

2. The ophthalmic apparatus of claim 1, wherein the one or more black spot plates are positioned at positions where the illumination light reflected by the objective lens is imaged in the first scanning optical system with the two or more illumination openings or the single illumination opening as object point.

3. The ophthalmic apparatus of claim 1, or 2, wherein
the light source unit includes:
a light source; and
a slit diaphragm in which a slit-shaped opening capable of being positioned at a position substantially conjugate optically to the photographed site is formed, wherein
the light source unit is configured to emit slit-shaped illumination light by irradiating light from the light source onto the slit diaphragm.

4. The ophthalmic apparatus of claim 1, 2, or 3, wherein in the dividing filter, the single light receiving opening is formed in a central region including a position corresponding to an optical axis of the illumination light, and the two or more illumination openings are formed in a peripheral region eccentric to the optical axis.

5. The ophthalmic apparatus of any one of claims 1 to 4, wherein in the dividing filter, the single illumination opening is formed in a central region including a position corresponding to an optical axis of the illumination light, and the two or more light receiving openings are formed in a peripheral region eccentric to the optical axis.

6. The ophthalmic apparatus of any one of claims 1 to 6, wherein the photographed site is a fundus.

7. The ophthalmic apparatus of any one of claims 1 to 6, wherein the one or more black spot plates are positioned at at least one of a position where the illumination light reflected on an anterior surface of the objective lens is imaged in the first scanning optical system, or a position where the illumination light reflected on a posterior surface of the objective lens is imaged in the first scanning optical system.

8. The ophthalmic apparatus of any one of claims 1 to 7, wherein the objective lens includes one or more doublets configured by sticking two or more lenses, and
the one or more black spot plates are positioned at positions where the illumination light reflected on one or more sticking surfaces of the one or more doublets is imaged in the first scanning optical system.

9. The ophthalmic apparatus of any one of claims 1 to 8, further comprising a deflecting element positioned at a position substantially conjugate optically to the pupil, and configured to deflect the returning light passing through the light receiving opening toward the descanning optical system.

10. The ophthalmic apparatus of any one of claims 1 to 9, wherein the descanning optical system includes a confocal diaphragm positioned at a position substantially conjugate optically to the photographed site.

11. The ophthalmic apparatus of any one of claims 1 to 10, further comprising a second scanning optical system positioned between the dividing filter and the imaging element, and configured to deflect the returning light to move a light-receptive region on an imaging surface of the imaging element.

12. The ophthalmic apparatus of claim 11, wherein
the scanning mirror is configured to deflect the illumination light and returning light of the illumination light that is guided through the objective lens on a first deflected surface, and
the second scanning optical system is configured to deflect the returning light passing through the light receiving opening on a second deflected surface that is backside of the first deflected surface of the scanning mirror.

13. The ophthalmic apparatus of claim 11, or 12, further comprising an image forming unit configured to form an image of the eye to be examined based on light receiving result of the imaging element read out using rolling shutter method.

14. The ophthalmic apparatus of any one of claims 1 to 13, further comprising an image correction unit configured to correct a black spot shadow depicted in the image, by performing luminance correction on the image of the eye to be examined acquired based on the light receiving result of the imaging element.

15. The ophthalmic apparatus of claim 14, wherein the image correction unit is configured to perform the luminance correction on at least one of two or more black spot shadows depicted in the image.

16. The ophthalmic apparatus of claim 15, wherein the image correction unit is configured to correct the black spot shadow by changing luminance in a predetermined region in the image by a predetermined amount.
